# EUROPEAN PATENT SPECIFICATION

(11) **EP 0 528 977 B1**
(45) Date of publication and mention of the grant of the patent: **02.11.1995**
(21) Application number: 91910663.3
(22) Date of filing: 14.05.1991
(51) Int. Cl.: A61M 5/00, A61M 1/00

(54) **FLOW CONTROL DEVICE HAVING SELECTABLE ALTERNATIVE FLUID PATHWAYS**
VORRICHTUNG ZUR DURCHFLUSSTEUERUNG MIT WÄHLBAREN, ALTERNATIVEN STRÖMUNGSWEGEN
DISPOSITIF DE REGULATION D'ECOULEMENT A CHEMINS DE FLUIDE ALTERNES SELECTIONNABLES

(30) Priority: 15.05.1990 US 524136; 02.05.1991 US 694896
(43) Date of publication of application: 03.03.1993
(73) Proprietor: PUDENZ-SCHULTE MEDICAL RESEARCH CORPORATION, Goleta, CA 93117 (US)
(72) Inventor: WATSON, David, A., Goleta, CA 93117 (US); EAST, Gary, P., Santa Barbara, CA 93110 (US)
(74) Representative: Ruschke, Hans Edvard, Dipl.-Ing.
(86) International application number: PCT/US91/03402
(87) International publication number: WO 91/17779

(56) References cited:
- GB-A- 2 054 802
- US-A- 3 985 140
- US-A- 4 560 375
- US-A- 4 621 654
- US-A- 4 741 730
- US-A- 4 761 158
- US-A- 4 781 673
- US-A- 4 781 674
- US-A- 4 795 437
- US-A- 4 850 955
- US-A- 4 861 331
- US-A- 4 867 740
- US-A- 4 867 741
- US-A- 4 995 864

## Description

### BACKGROUND OF THE INVENTION

This invention relates generally to surgically implanted physiological shunt systems and related flow control devices. More particularly, the present invention relates to shunt systems including one-way flow control valves for controlling the flow of cerebrospinal fluid out of a brain ventricle and preventing backflow of fluid into the brain ventricle.

In the medical arts, to relieve undesirable accumulation of fluids it is frequently necessary to provide a means for draining a fluid from one part of the human body to another in a controlled manner. This is required, for example, in the treatment of hydrocephalus, a ailment usually afflicting infants or children in which fluids accumulate within the skull and exert extreme pressure and skull deforming forces.

In treating hydrocephalus, cerebrospinal fluid accumulated in the brain ventricles is typically drained away utilizing a drainage or shunt system including a catheter inserted into the ventricle through the skull, which is connected to a tube which conducts the fluid away from the brain to be reintroduced into the peritoneal cavity or into the vascular system, as by extending a distal catheter through the patient's jugular vein to the atrium portion of the heart. To control the flow of cerebrospinal fluid and maintain the proper pressure in the brain ventricle, a pump or valve is placed in the conduit between the brain and the peritoneal cavity or the heart. An exemplary flow control device is found in U.S. Patent No. 4,560,375.

Although such drainage systems have provided successful results, a problem of over drainage of the cerebrospinal fluid from the brain ventricles sometimes exists. Over drainage of cerebrospinal fluid may result in excessive reduction of the cerebrospinal fluid pressure within the brain ventricles and predispose the development of a subdural hematoma or hydroma, and excessive reduction of ventricular size leading to shunt obstruction because of impingement of the ventricular walls on the inlet holes of the ventricular catheter. This over drainage can be caused by the siphoning effect of hydrostatic pressure in the distal shunt catheter. The siphoning effect of hydrostatic pressure may be created by the elevation of the ventricular catheter inlet with respect to the distal catheter outlet (i.e., when the patient sits, stands or is held erect). In order to prevent such over drainage caused by the siphoning effect of hydrostatic pressure in the distal shunt catheter, siphon control devices have been placed in the conduit, typically between the flow control device and the peritoneal cavity or the heart. An exemplary siphon control device is found in U.S. Patent No. 4,795,437.

It is desirable in some instances to permit the physician to be able to alter the flow characteristics through the drainage system after it has been subcutaneously implanted. To this end, on-off devices have been provided for implantation as a portion of the fluid conduit, as an additional element of the shunt. An exemplary on-off device is shown in U.S. Patent No. 3,827,439.

Furthermore, a flow control device according to the preamble of claim 1 is known from US-A-3 985 140.

Prior physiological shunt systems have failed to provide, however, a flow control device which permits only unidirectional flow through the shunt system, prevents over drainage caused by the siphoning effect of hydrostatic pressure in the distal shunt catheter, and permits the flow characteristics through the shunt to be altered by means of percutaneous pressure after the shunt system has been surgically implanted. Further, existing flow control devices extensively used in connection with the treatment of hydrocephalus typically provide only a single pre-set resistance to the flow of excess cerebrospinal fluid through the shunt system, which cannot be varied except to prevent fluid flow through the shunt by means of an on-off valve.

Accordingly, there has been a continuing need in the medical arts for convenient and effective physiological drainage devices for controlling the flow of fluid from one part of the body to another, which are relatively inexpensive to manufacture and can be constructed substantially of non-metallic parts which are not subject to adhering to one another and causing a malfunction of the device. A flow control device is needed which permits fluid flow therethrough only when upstream fluid pressure exceeds downstream fluid pressure by a selected pressure differential, and which also provides means for altering the selected pressure differential by manual percutaneous manipulation of the device when it is subcutaneously implanted.

Additionally, a novel flow control device for use in a physiological shunt system is needed which utilizes a plurality of flow control valves having different flow control characteristics. Such a device should provide alternative fluid pathways therethrough such that selection of the desired fluid pathway can be made by the selective application of percutaneous manual pressure to the device when it is subcutaneously implanted. Moreover, such a flow control device is needed which incorporates an integral siphon control device that opens only in response to positive upstream fluid pressure, and recloses or remains closed in the absence of such positive upstream fluid pressure or in response to negative downstream hydrostatic pressure on the device. As will become apparent from the following description, the present invention satisfies these needs and provides other related advantages.

### SUMMARY OF THE INVENTION

The present invention is defined by the features of the appended claim 1.

Further, advantageous embodiments of the present invention are defined in dependent claims 2 to 9.

### BRIEF DESCRIPTION OF THE DRAWINGS

The accompanying drawings illustrate the invention. In such drawings:
FIGURE 1 is a perspective view of a flow control device having selectable alternative fluid pathways, embodying the invention;
FIGURE 2 is an enlarged vertical section taken generally along the line 2-2 of FIG. 1;
FIGURE 3 is a horizontal section taken taken generally along the line 3-3 of FIG. 2, illustrating the configuration of the flow control device when the second fluid conduit is open to fluid flow;
FIGURE 4 is an enlarged vertical section taken generally along the line 4-4 of FIG. 2, illustrating the configuration of a plug which is positioned so as not to occlude the second fluid conduit;
FIGURE 5 is a vertical sectional view similar to that illustrated in FIG. 2, illustrating the manner in which a portion of the fluid flow path adjacent to an inlet can be occluded by manual percutaneous pressure, and the manner in which additional manual percutaneous pressure can be applied to a portion of the housing to flush fluid through the device;
FIGURE 6 is a vertical sectional view similar to FIG. 4, illustrating the manner in which the plug can be inserted into a collar defining a portion of the second fluid conduit, to occlude the second fluid conduit to fluid flow therethrough; and
FIGURE 7 is a horizontal sectional view similar to FIG. 3, illustrating, in fragmented form, a portion of the flow control device and the manner in which fluid flows past two normally closed valves when the second fluid conduit is occluded.

### DETAILED DESCRIPTION OF THE PREFERRED EMBODIMENT

As shown in the drawings for purposes of illustration, the present invention is concerned with an improved flow control device, generally designated in the accompanying drawings by reference number 10. The improved flow control device 10 is intended for use in a surgically implanted physiological shunt system for draining fluid from one portion of the body to another. In order to connect, for example, the device 10 in such a system, the device includes an inlet connector 12 and an outlet connector 14 which each receive one end of a piece of surgical tubing (not shown). The ends of the surgical tubing are placed over the connectors 12 and 14 and secured thereon by a single ligature just inside of an annular ridge 16 formed near the end of each connector.

When the flow control device 10 is used in a drainage system intended for the treatment of hydrocephalus, the inlet connector 12 is fluidly connected with a proximal catheter which is inserted through the skull into a brain ventricle containing cerebrospinal fluid under pressure. The outlet connector 14 is fluidly connected to a distal catheter which serves to discharge cerebrospinal fluid into, for example, the atrium portion of a patient's heart. Ordinarily the flow control device 10 will be surgically implanted on the patient's skull with a flap of skin overlying the device. To facilitate holding the device in its desired position after implantation, a generally flexible mounting plate 18 can be provided with one or more suture holes.

As will become apparent from the following description, the present invention provides a highly reliable flow control device having selectable alternative fluid pathways which permit the flow control characteristics of the device 10 to be altered when subcutaneously implanted by manual percutaneous manipulation of the device. The present invention provides a highly reliable flow control device designed to prevent valve seat deformation and membrane to valve seat sticking, and to facilitate implantation by eliminating components to be connected or adjusted other than the surgical tubing to the device itself.

In accordance with the present invention, the flow control device 10 includes a pair of relatively rigid, molded plastic bases 20 and 22 invested within a resiliently flexible housing 24 which, together, define a fluid flow path through the housing from the inlet connector 12 to the outlet connector 14. Two normally closed valves 26 and 28 are provided within the fluid flow path to restrict the flow of fluid through the device 10. The housing 24 and the second base 22 cooperate to provide a siphon control device 30, situated between the valves 26 and 28 and the outlet connector 14, which prevents fluid flow through the device 10 in the absence of positive upstream fluid pressure or in response to negative downstream hydrostatic pressure on the device. Further, the housing 24 and the first base 20 cooperate to define a pump or flushing reservoir 32 between the inlet connector 12 and the valves 26 and 28.

Two alternative fluid conduits, defining portions of the fluid flow path through the device 10, are provided in order to permit the flow control characteristics of the device 10 to be altered by manual percutaneous pressure applied to the device when subcutaneously implanted. A first fluid conduit 34 directs fluid through both the first and second normally closed valves 26 and 28. A second fluid conduit 36 bypasses the first normally closed valve 26 and directs fluid through the second normally closed valve 28 only.

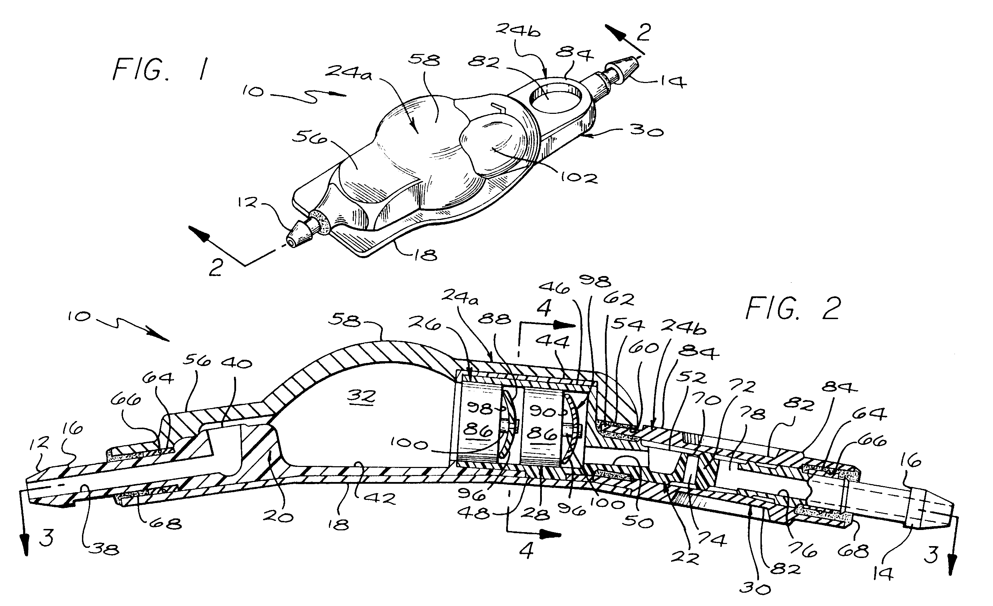

More specifically, and as shown best in FIGS. 2-7, the bases 20 and 22 interfit with one another and are integrally formed with, respectively, the inlet and outlet connectors 12 and 14. The first base 20 is integrally formed with the inlet connector 12 and defines an inlet flow channel 38 extending the inlet connector 12 to an upwardly facing inlet occluder port 40. The first base 20 further forms a bottom plate 42 for the flushing reservoir 32, and a generally cylindrical receptacle 44 which receives a portion of the second base 22.

The second base 22 is integrally formed with the outlet connector 14 and includes a generally cylindrical valve housing 46 which is received, in an interference fit, into the receptacle 44 of the first base 20. The valve housing 46 includes a housing key 48 which snap-fits into an aperture provided through the receptacle 44, for the purpose of properly aligning the second base 22 with respect to the first base 20. The second base 22 defines a valve housing outlet flow channel 50 which terminates at an upwardly facing outlet occluder port 52. Between the outlet occluder port 52 and the outlet connector 14, the second base 22 defines a portion of the siphon control device 30.

The flexible housing 24 is provided in two parts: an inlet housing portion 24a in which the first base 20 and a portion of the second base 22 are invested, and an outlet housing portion 24b which is sealed by a suitable adhesive 54 to the inlet housing portion 24a in order to provide a continuous elastomeric exterior to the device 10, with the exception of the inlet and outlet connectors 12 and 14 which extend therefrom. The inlet housing portion 24a is integrally formed with the mounting plate 18 and includes an inlet aperture through which the inlet connector 12 extends, an inlet occluder wing 56 which generally overlies the inlet occluder port 40, and a resiliently flexible dome 58 for the flushing reservoir 32. The inlet housing 24a surrounds the receptacle 44 and adjacent portions of the second base 22, and includes a recess 60 in which an engaging flange 62 of the outlet housing 24b is secured by means of the adhesive 54.

In order to provide a fluid-tight seal between the inlet connector 12 and the housing 24a, a tube 64 is placed over a portion of the inlet connector and secured in place by means of an over suture 66. A silicone adhesive 68 is then injected into the remaining gap between the housing 24a and the inlet connector 12. This same sealing arrangement is made between the housing 24b and the outlet connector 14.

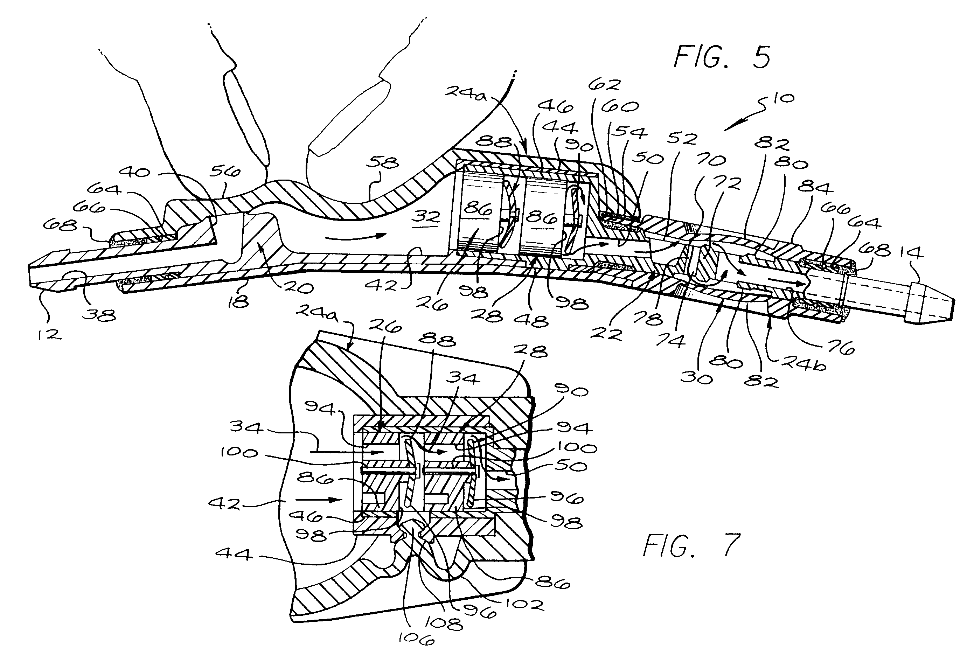

The inlet occluder wing 56 is positioned over the inlet occluder port 40 to facilitate occluding the inlet flow channel 38 at the port 40 by pressing the wing 56 downwardly. Depressing the wing 56 and occluding the port 40 prevents proximal fluid flow from the flushing reservoir 32, defined by the dome 58 and the bottom plate 42, when the dome is pressed downwardly by manual percutaneous pressure (see FIG. 5). The dome 58 is preferably molded of a silicone elastomer material and is designed to permit injection into the flow control device 10 by a hypodermic needle through the dome. The bases 20 and 22 are preferably molded of a polypropylene material which provides sufficient rigidity to prevent a needle from inadvertently passing through the device 10 if an injection is made into the flushing reservoir 32. The construction of the bases 20 and 22 and the housing 24 helps to guide a physician in manually percutaneously manipulating the device 10 when subcutaneously implanted, for purposes of flushing the shunt system and/or altering the flow characteristics of the flow control device.

The outlet housing portion 24b surrounds a portion of the second base 22 to define the siphon control device 30 which is similar to that shown and described in U.S. Patent No. 4,795,437, the contents of which are incorporated herein by reference. The siphon control device 30 includes an outer wall 70 and an inner wall 72 which is situated within and encircled about by the outer wall. The valve housing outlet flow channel 50 channels fluid from the second normally closed valve 28 to a central SCD reservoir 74 defined as the area between the inner wall 72 and the outer wall 70. An outlet flow channel 76 extends through the inner wall 72 to the distal end of the outlet connector 14.

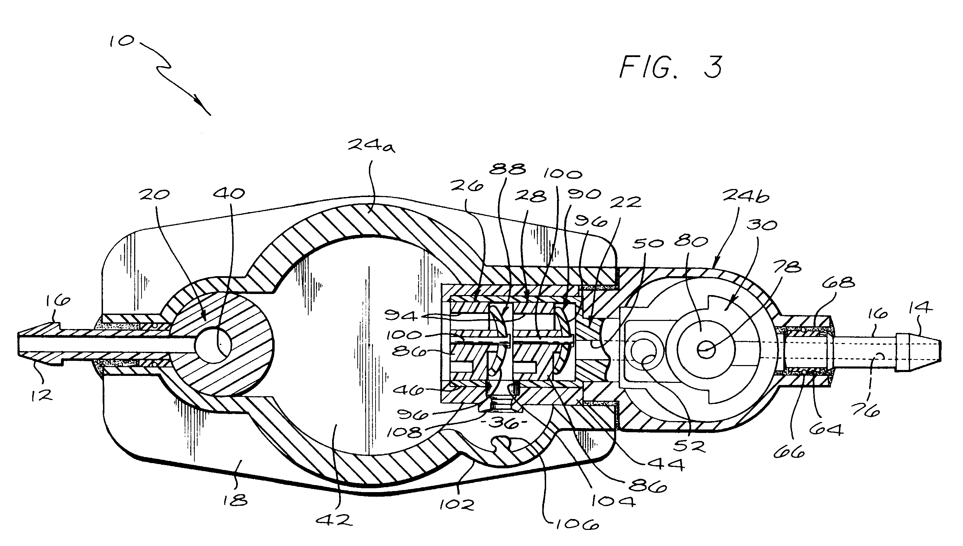

As can be seen best in FIG. 3, the outer wall 70 is generally circular in shape, and is spaced from and encircles the inner wall 72. The inner wall is also generally circular in shape, and defines an outlet chamber 78 which is adjacent to and in fluid communication with the outlet flow channel 76. The inner wall 72 is constructed to have substantially parallel upper and lower seating surfaces 80, and it effectively forms a barrier separating the SCD reservoir 74 from the outlet chamber 78.

The outlet housing portion 24b is provided with a pair of spaced, substantially parallel, flexible and elastic diaphragms 82 which are fixed about their peripheries adjacent to the outer wall 70. Each diaphragm has an inner surface which defines the upper and lower limits of the SCD reservoir 74 and the outlet chamber 78, and an outer surface which forms an exterior surface of the siphon control device 30. The diaphragms 82 are situated on opposite sides of the inner wall 72 to position a portion of each inner surface thereof in contact with an adjacent one of the seating surfaces 80 and form a seal therebetween which prevents fluid flow between the outlet occluder port 52 and the outlet flow channel 76.

The outlet housing portion 24b further includes integral offset rings 84 which surround each diaphragm 82 to inhibit overlying tissue from occluding the siphon control device 30 when implanted into a patient. An aperture is provided through the housing 24b through which the outlet connector 14 extends. A fluid tight seal is effected between the housing outlet aperture and the outlet connector 14 utilizing a tube 64, an over suture 66 and an adhesive 68, as described above in connection with the inlet housing portion 24a and the inlet connector 12.

In use, the diaphragms 82 normally lie against and interact with the seating surfaces 80 of the inner wall 72 to close the device 10 to fluid flow. The diaphragms 82 move away from the seating surfaces 80, however, in response to a minimal level of positive fluid pressure within the SCD reservoir 74 to permit passage of fluid from the valve housing flow channel 50 to the outlet flow channel 76 (see FIG. 5). The diaphragms 82 close and seal upon the seating surfaces 80 once again in the absence of such positive upstream fluid pressure, or in response to negative downstream hydrostatic pressure in the outlet chamber 78. The siphon control device 30 thus minimizes the undesirable consequences attendant to excessive over drainage of fluid due to the siphoning effect of hydrostatic pressure.

With reference now to the normally closed valves 26 and 28, each valve includes a valve membrane carrier 86 which is securely positioned within the valve housing 46 of the second base 22. Each valve membrane carrier 86 provides a cylindrical support for a respective flow control member 88 or 90, and is press-fit within the valve housing 46.

Each of the valve membrane carriers 86 includes a cylindrical outer portion which abuts against a correspondingly shaped inner surface of the valve housing 46 to form a fluid tight seal therebetween. A central reinforced aperture 92 is provided for receiving a portion of the respective flow control member 88 or 90, and the central aperture 92 is surrounded by three additional apertures 94 which provide a plurality of channels through which fluid is permitted to flow.

The valves 26 and 28 are arranged serially within the valve housing 46 for controlling the flow of cerebrospinal fluid out of a brain ventricle. Each flow control member 88 and 90 includes a membrane 96, molded of a synthetic polymer material different from the material of the valve membrane carrier 86, which is secured relative to the valve membrane carriers to generally cover the apertures 94.

The resilient membranes 96 are normally biased to close communication between inlet and outlet sides of each valve 26 and 28, but will open to permit flow when the pressure on the inlet or proximal side of the resilient membrane exceeds the pressure on the outlet or distal side by a predetermined amount. Moreover, should the pressure on the distal side of either resilient membrane 96 ever exceed the pressure on the proximal side, tending to cause flow in a reverse direction through the valves 26 and 28, the membrane 96 will seal tightly against a valve seat 98 provided on the valve membrane carrier 86, to prevent any such reverse fluid flow.

The valve membrane carriers 86 are preferably formed of a polypropylene material, and the membranes 96 are preferably formed of a silicone elastomer material. Both polypropylene and elastomer materials have been shown to produce an acceptable level of tissue reaction, and the use of this particular duality of materials, in contrast to the use of only a single material, markedly decreases the chance of the membranes 96 adhering to any portion of the valve membrane carriers 86, which would clog the drain passage and defeat the purpose of the valves 26 and 28.

An added advantage of using these particular materials is the avoidance of the negative effect of metal components, due to radiation scatter or "sunburst effect," on films taken by, for example, computerized axial tomography (CAT) scanning equipment. This type of scanning frequently accompanies the use of surgically implanted flow control valves, and the absence or limitation of metal in the areas scanned will permit more accurate and complete results to be gathered from CAT scanning.

The flow control members 88 and 90 each include a rigid nail 100 having an elongated shaft and an expanded head at an end of the shaft. The nail 100 is capable of being driven into the central aperture 92 of the respective valve membrane carrier 86 to be frictionally retained therein. The nail 100 is preferably constructed of a polypropylene material which will not stretch or flex as the nail is being driven into the central aperture 92. After the nail is properly placed within the aperture, it is preferably heat welded in place.

The membranes 96 have an arch-shape, as for example a section of a sphere, and are designed to contact the valve seats 98 generally along the outer edges of the membrane in a manner surrounding the apertures 94. Each membrane 96 is secured to its respective valve membrane carrier 86 by the central support/nail 100. The membrane is retained in place on the nail by an interference fit or by use of an adhesive, or by any other suitable means.

Since the valves 26 and 28 are primarily designed to provide controlled resistance to cerebrospinal fluid flow from a brain ventricle to another location in the body, it will be appreciated that a doctor must be able to select valves having the particular pressure/flow characteristics desired for each individual application. That is, a valve which permits flow at a relatively low pressure differential may not be suitable where the maintenance of a higher pressure differential is indicated. Toward this end, in order to provide a flow control device with a variety of different pressure/flow characteristics, the first normally closed valve 26 has a different pressure/flow characteristic than the second normally closed valve 28. More particularly, the first normally closed valve 26 is provided with a relatively thick membrane 96, whereas the second normally closed valve 28 is provided a relatively thin membrane 96. Resistance to flow increases with an increase in membrane thickness. Thus, the first normally closed valve provides a higher degree of resistance to flow through the device 10 than the second normally closed valve 28.

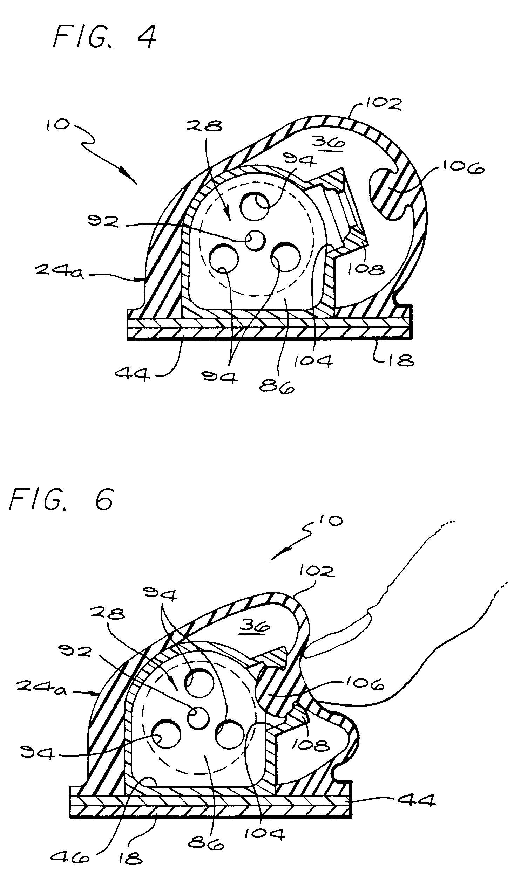

Recognizing that fluid will tend to take the path of least resistance, it can be seen that fluid permitted to flow through the second fluid conduit 36, thereby bypassing the first normally closed valve 26, will experience a lesser degree of resistance to flow than if forced to flow through the first fluid conduit 34. FIGS. 1 through 4 illustrate the configuration of the flow control device 10 wherein the second fluid conduit 36 is open to fluid flow therethrough. The inlet housing portion 24a includes a bypass portion 102 which overlies aligned apertures 104 provided through both the receptacle 44 and the valve housing 46. As shown best in FIGS. 3 and 4, fluid is permitted to flow from the flushing reservoir 32 between the bypass portion 102 and the first base 20, and through the aligned apertures 104 for introduction on the inlet side of the second normally closed valve 28. Since both of the normally closed valves 26 and 28 prevent retrograde fluid flow, the fluid passes only through the second normally closed valve 28 into the valve housing outlet flow channel 50. Accordingly, resistance to flow through the flow control device 10 when the second fluid conduit 36 is open, is determined primarily by the second normally closed valve 28. From the flow channel 50, the fluid then passes through the siphon control device 30 to the outlet flow channel 76, for delivery to a distal catheter.

Integrally formed with the bypass portion 102 is a plug 106 configured to occlude the aligned apertures 104. The receptacle 44 includes a collar 108 designed to retain the plug 106 therein (FIGS. 6 and 7) when inserted by manual percutaneous pressure applied to the bypass portion 102 of the housing 24a. With the plug 106 held in place by the collar 108 to occlude the aligned apertures 104, fluid flow through the second fluid conduit 36 is prevented, thereby requiring any fluid flow through the flow control device 10 to occur solely through the first fluid conduit 34.

Fluid flow through the first fluid conduit 34 is illustrated best in FIG. 7, which shows that fluid within the flushing reservoir 32 must pass through both the first and second normally closed valves 26 and 28. Since the first normally closed valve 26 provides a greater degree of resistance to flow than the second normally closed valve 28, the first valve 26 primarily determines the resistance to flow of the device 10 in this configuration.

The design of the flow control device 10, as described above, lends itself to easy percutaneous distal and proximal flushing, and permits the selective adjustment of flow characteristics by percutaneous manual manipulation when subcutaneously implanted. In order to flush the device 10 distally, the inlet occluder wing 56 is pressed downwardly to occlude the inlet occluder port 40. The dome 58 is then simply pressed downwardly (FIG. 5) to flush the contents of the flushing reservoir 32 through the second fluid conduit 36 (or the first fluid conduit 34 if the second fluid conduit is occluded), past the siphon control device 30 and through the outlet flow channel 76. Similarly, proximal flushing can be accomplished by pressing downwardly the offset ring 84 of the siphon control device 30 adjacent to the distal end of the inlet housing portion 24a, to occlude the outlet occluder port 52. The dome 58 is then pressed downwardly to flush fluid proximally from the flushing reservoir 32 out of the device 10 through the inlet flow channel 38.

In order to dislodge the plug 106 from the collar 108 and thereby open the second fluid conduit 36, pressure is applied to the housing 24 to occlude both the inlet occluder port 40 and the outlet occluder port 52, and then the dome 58 is depressed as if to flush the device 10. However, since both the distal and proximal flow paths for fluid within the flushing reservoir 32 are occluded, the static fluid pressure between the occluder ports 40 and 52 is raised sufficiently to dislodge the plug 106 from the collar 108. The housing 24 is preferably formed of a resiliently flexible material so that after dislodging the plug 106 from the collar 108, the bypass portion 102 of the housing takes on its original shape to permit fluid flow through the aligned apertures 104 of the second fluid conduit 36.

From the foregoing it is to be appreciated that the present invention provides a flow control device 10 for use in a subcutaneously implanted physiological shunt system having selectable alternative means for controlling fluid flow through the fluid flow path. Through the provision of a first fluid conduit 34 which directs fluid through both the first and second normally closed valves 26 and 28, and an alternative second fluid conduit 36 which bypasses the first normally closed valve 26, means are provided for permitting fluid flow when upstream fluid pressure exceeds downstream fluid pressure by two different selected pressure differentials. The construction of the flow control device 10 of the present invention permits selective distal and proximal flushing of the device through the application of manual percutaneous pressure, and further permits the selected pressure differential to be altered, also through percutaneous manual manipulation of the device when subcutaneously implanted. The present invention provides a device by which the flow of cerebrospinal fluid out of a brain ventricle can be controlled while preventing the backflow of fluid into the brain ventricle, and inhibits excessive drainage through the physiological shunt in the presence of excessive downstream suction.

Although a particular embodiment of the invention has been described in detail for purposes of illustration, various modifications may be made without departing from the scope of the invention as defined by the appended claims.

## Claims

1. A flow control device having selectable alternative fluid pathways, for use in a subcutaneously implanted physiological shunt system, the flow control device comprising:
a) a housing having an inlet (12) and an outlet (14), and first and second valves (26;28) between said inlet and outlet for controlling fluid flow from the inlet to the outlet;
b) first fluid conduit means (34) for directing fluid through the first (26) and second (28) valves as the fluid passes through the flow control device;
c) second fluid conduit means (36) for bypassing the first valve (26) and directing fluid through the second valve (28) as the fluid passes through the flow control device; and
d) means for selectively directing fluid either through the first (34) or second (36) fluid conduit means, the means for selectively directing fluid including percutaneously actuable means for selectively occluding and, alternatively, opening the second fluid conduit means (36),
e) a flushing reservoir,
characterized in that
- the flushing reservoir (32) is adjacent to the inlet (12), has an overlying, resiliently deformable dome (58) forming a portion of the housing, the flushing reservoir providing means for flushing fluid through the flow control device by application of percutaneous manual pressure to the device to depress the dome (58); and
- said first valve (26) is situated between the flushing reservoir (32) and the outlet (14), and said second valve (28) is situated between the first valve (26) and the outlet (14).

2. A flow control device as set forth in claim 1, wherein the first and second valves (24;26) each include a base (86) within the housing having a valve passageway (94) therethrough and a valve seat (98) surrounding a portion of the valve passageway, and a flow control member (88;90) attached to the base and contacting the valve seat in a manner normally occluding the valve passageway but selectively opening to permit controlled unidirectional flow therethrough.

3. A flow control device as set forth in claim 2, wherein each flow control member (88;90) includes a rigid nail-like central support (100) and a resilient membrane (96), the central support being attached to the respective base (86) and extending therefrom to support the resilient membrane, the resilient membrane being generally arch-shaped and having a portion thereof capable of engaging the respective valve seat (98) to occlude the respective fluid passageway.

4. A fluid flow control device as set forth in claim 3, wherein the first valve (26) provides a greater resistance to flow than the second valve (28).

5. A flow control device as set forth in claim 1, wherein the means for occluding the second fluid conduit means (36) includes plug means (106) integrally formed with the housing and insertable into the second fluid conduit means (36) by manual percutaneous pressure applied to the device, which plug means is retained in place to occlude the second fluid conduit means until displaced therefrom by the selective application of manual percutaneous pressure to the device.

6. A flow control device as set forth in claim 1, including a siphon control device (30) adjacent to the outlet (14), which prevents fluid flow through the flow control device in the absence of positive upstream fluid pressure through the device or in response to negative hydrostatic downstream pressure on the device, the siphon control device providing means for occluding the outlet by application of percutaneous manual pressure to the device.

7. A fluid flow control device as set forth in claim 6, wherein the siphon control device (30) comprises a control housing including a pair of spaced, substantially parallel flexible diaphragms (82) having inner and outer surfaces, and top and bottom rings (84) wherein each is positioned to surround a respective diaphragm to provide means for inhibiting overlying tissue from occluding the device, and a base invested within the control housing, the base including a wall (72) having an inlet side and an outlet side, and substantially parallel upper and lower seating surfaces (80).

8. A fluid flow control device as set forth in claim 1, including means for temporarily occluding the inlet (12) by application of percutaneous manual pressure to the housing such that depression of the dome (58) flushes fluids distally through the device.

9. A fluid flow control device as set forth in claim 8, including means for temporarily occluding the outlet (14) by application of percutaneous manual pressure to the housing such that depression of the dome (58) flushes fluids proximally through the device.

## Patentansprüche

1. Durchflußsteuereinrichtung mit wählbaren alternativen Durchflußwegen für eine Flüssigkeit zum Einsatz in einem subkutan implantierten physiologischen Shuntsystem, die
a) ein Gehäuse mit einem Zulauf (12) und einem Ablauf (14) sowie ein erstes und ein zweites Ventil (26; 28) zwischen dem Zu- und dem Ablauf zum Steuern des Durchflusses vom Zu- zum Ablauf,
b) einen ersten Flüssigkeitskanal (34), der Flüssigkeit beim Durchfließen der Durchflußsteuereinrichtung durch das erste und das zweite Ventil (26; 28) richtet,
c) einen zweiten Flüssigkeitskanal (36) zum Umgehen des ersten Ventils (26) und zum Richten der Flüssigkeit durch das zweite Ventil (28) beim Durchfluß derselben durch die Durchflußsteuereinrichtung,
d) Mittel zum wahlweisen Richten von Flüssigkeit durch entweder den ersten (34) oder den zweiten (36) Flüssigkeitskanal, einschließlich von perkutan betätigbaren Mitteln zum wahlweisen Verschließen und alternativen Öffnen des zweiten Flüssigkeitskanals (36), sowie
e) ein Spülreservoir aufweist,
**dadurch gekennzeichnet,** daß
- das Spülreservoir (32) sich am Zulauf (12) befindet, eine überlagernde, federelastisch verformbare Kuppe (58) einen Teil des Gehäuses bildet und Mittel schafft, um durch perkutane Beaufschlagung von Hand zum Herabdrücken der Kuppe (58) die Durchflußsteuereinrichtung mit Flüssigkeit durchzuspülen, und
- das erste Ventil (26) zwischen dem Spülreservoir (32) und dem Ablauf (14) und das zweite Ventil (28) zwischen dem ersten Ventil (26) und dem Ablauf (14) liegen.

2. Durchflußsteuereinrichtung nach Anspruch 1, bei der das erste und das zweite Ventil (24; 26) jeweils einen Basisteil (86) im Gehäuse mit einem Ventilkanal (94) und einem einen Teil des Ventilkanals umgebenden Ventilsitz (98),sowie ein Durchflußsteuerelement (88; 90) aufweist, das am Basisteil befestigt ist und den Ventilsitz auf eine Weise kontaktiert, bei der der Ventilkanal normalerweise gesperrt wird, aber wahlweise öffnet, um eine kontrollierte Strömung in nur einer Richtung zu ermöglichen.

3. Durchflußsteuereinrichtung nach Anspruch 2, bei der jedes Strömungssteuerelement (88; 90) einen starren nagelartigen mittigen Träger (100) und eine federelastische Membran (96) aufweist, der mittige Träger am jeweiligen Basisteil (86) befestigt ist und von diesem absteht, um die federlelastische Membran zu tragen, und die federelastische Membran allgemein gewölbt ist und einen Teil aufweist, der sich an den jeweiligen Ventilsitz (98) anlegen kann, um den entsprechenden Flüssigkeitskanal zu sperren.

4. Durchflußsteuereinrichtung nach Anspruch 3, bei der das erste Ventil (26) einer Strömung einen höheren Widerstand entgegensetzt als das zweite Ventil (28).

5. Strömungssteuereinrichtung nach Anspruch 1, bei der die Einrichtung zum Sperren des zweiten Kanals (36) einen einteilig mit dem Gehäuse gebildeten Stopfen (106) aufweist, der durch von Hand perkutan auf die Einrichtung aufgebrachten Druck in den zweiten Flüssigkeitskanal (36) einsteckbar ist, wobei der Stopfen in der den zweiten Flüssigkeits sperrenden Lage verbleibt, bis er durch wahlweise Beaufschlagung der Einrichtung mit perkutanem Druck von Hand gelöst wird.

6. Durchflußsteuereinrichtung nach Anspruch 1 mit einer Siphonsteuereinrichtung (30) am Ablauf (14), die bei fehlendem stromaufwärtigem Flüssigkeits-Überdruck in der Einrichtung oder bei stromabwärtigem hydrostatischem Unterdruck in der einrichtung eine Strömung durch die Durchflußsteuereinrichtung verhindert, wobei die Siphonsteuereinrichtung Mittel zum Sperren des Ablaufs durch Beaufschlagen der Einrichtung mit perkutanem Druck von Hand schafft.

7. Durchflußsteuereinrichtung nach Anpruch 6, bei der die Siphonsteuereinrichtung (30) ein Gehäuse mit einem Paar beabstandeter, im wesentlichen paralleler flexibler Membranen (82) mit einer Innen- und einer Außenfläche sowie einem oberen und unteren Ring (84) aufweist, die jeweils eine zugehörige Membran umgebend angeordnet sind, um ein Mittel zu schaffen, überlagerndes Gewebe am Zusetzen der Einrichtung zu hindern, und bei der im Gehäuse ein Basisteil mit einer Wandung (72) mit einer Zu- und einer Ablaufseite sowie oberen und unteren Sitzfläche (80) aufweist, die im wesentlichen parallel liegen.

8. Durchflußsteuereinrichtung nach Anspruch 1 mit Mitteln zum zeitweiligen Sperren des Ablaufs (12) durch Beaufschlagen des Gehäuses mit perkutanem Druck von Hand derart, daß durch Eindrücken der Kuppe (58) Flüssigkeit distal durch die Einrichtung gedrückt wird.

9. Durchflußsteuereinrichtung nach Anspruch 8 mit Mitteln zum zeitweiligen Sperren des Ablaufs (14) durch Beaufschlagen des Gehäuses mit perkutanem Druck von Hand derart, daß durch Eindrücken der Kuppe (58) Flüssigkeit proximal durch die Einrichtung gedrückt wird.

## Revendications

1. Appareil de réglage de circulation ayant des trajets alternés de fluide qui peuvent être choisis, destiné à être utilisé dans un circuit de dérivation physiologique implanté sous la peau, l'appareil de réglage de circulation comprenant :
a) un boîtier ayant une entrée (12) et une sortie (14), et une première et une seconde soupape (26 ; 28) placées entre l'entrée et la sortie et destinées à régler la circulation du fluide de l'entrée à la sortie,
b) un premier conduit (34) de fluide destiné à diriger le fluide dans la première (26) et la seconde (28) soupape lorsque le fluide circule dans l'appareil de réglage de circulation,
c) un second conduit de fluide (36) destiné à passer en dérivation par rapport à la première soupape (26) et à diriger le fluide dans la seconde soupape (28) lorsque le fluide circule dans l'appareil de réglage de circulation,
d) un dispositif destiné à diriger sélectivement le fluide soit dans le premier conduit (34) soit dans le second (36), le dispositif destiné à diriger sélectivement le fluide comprenant un dispositif qui peut être commandé de manière percutanée pour une occlusion sélective ou au contraire une ouverture du second conduit de fluide (36), et
e) un réservoir d'entraînement,
caractérisé en ce que :
- le réservoir d'entraînement (32) est adjacent à l'entrée (12), et possède un dôme élastiquement déformable (58) qui le recouvre et qui constitue une partie du boîtier, le réservoir d'entraînement formant un dispositif d'entraînement du fluide dans l'appareil de réglage de circulation par application d'une pression manuelle percutanée à l'appareil afin que le dôme (58) sont enfoncé, et
- la première soupape (26) est placée entre le réservoir d'entraînement (32) et la sortie (14), et la seconde soupape (28) est placée entre la première soupape (26) et la sortie (14).

2. Appareil de réglage de circulation selon la revendication 1, dans lequel la première et la seconde soupape (24 ; 26) comportent chacune une base (86) placée dans le boîtier et ayant un passage (94) de soupape qui la traverse et un siège (98) de soupape qui entoure une partie du passage de soupape, et un organe (88 ; 90) de réglage de circulation fixé à la base et placé au contact du siège de soupape d'une manière qui bouche normalement le passage de soupape mais qui l'ouvre sélectivement pour permettre une circulation unidirectionnelle réglée.

3. Appareil de réglage de circulation selon la revendication 2, dans lequel chaque organe de réglage de circulation (88 ; 90) comprend un support central rigide (100) en forme de clou et une membrane élastique (96), le support central étant fixé à la base respective (86) et dépassant de celle-ci pour le support de la membrane élastique, la membrane élastique ayant de façon générale une forme bombée et comprenant une partie qui peut être au contact du siège respectif (98) de soupape afin qu'elle bouche le passage respectif de fluide.

4. Appareil de réglage de circulation de fluide selon la revendication 3, dans lequel la première soupape (26) introduit une plus grande résistance à la circulation que la seconde soupape (28).

5. Appareil de réglage de circulation selon la revendication 1, dans lequel le dispositif de bouchage du second conduit (36) comporte un bouchon (106) formé en une seule pièce avec le boîtier et destiné à pénétrer dans le second conduit de fluide (36) sous l'action d'une pression manuelle percutanée appliquée à l'appareil, le bouchon étant maintenu en place afin qu'il bouche le second conduit de fluide jusqu'à ce qu'il en soit chassé par application sélective d'une pression percutanée manuelle à l'appareil.

6. Appareil de réglage de circulation selon la revendication 1, comprenant un appareil (30) de réglage à siphon adjacent à la sortie (14) et qui empêche la circulation du fluide dans l'appareil de réglage de circulation en l'absence d'une pression positive en amont dans l'appareil ou en présence d'une pression hydrostatique négative en aval de l'appareil, l'appareil de réglage à siphon constituant un dispositif destiné à boucher la sortie par application d'une pression manuelle percutanée à l'appareil.

7. Appareil de réglage de circulation d'un fluide selon la revendication 6, dans lequel l'appareil (30) de réglage à siphon comporte un boîtier de commande contenant deux diaphragmes flexibles distants et sensiblement parallèles (82) ayant des surfaces interne et externe, et des anneaux supérieur et inférieur (84), chaque anneau étant destiné à entourer un diaphragme respectif afin qu'il forme un dispositif destiné à empêcher l'occlusion de l'appareil par les tissus placés sur lui, et une base logée dans le boîtier de commande, la base ayant une paroi (72) ayant un côté d'entrée et un côté de sortie et des surfaces supérieure et inférieure pratiquement parallèles de siège (80).

8. Appareil de réglage de circulation de fluide selon la revendication 1, comprenant un dispositif de bouchage temporaire de l'entrée (12) par application d'une pression manuelle percutanée au boîtier afin que l'enfoncement du dôme (58) chasse les fluides du côté distal dans l'appareil.

9. Appareil de réglage de circulation de fluide selon la revendication 8, comprenant un dispositif destiné à boucher temporairement la sortie (14) par application d'une pression manuelle percutanée au boîtier afin que l'enfoncement du dôme (58) chasse les fluides du côté proximal dans l'appareil.
